# EUROPÄISCHE PATENTSCHRIFT

(11) **EP 1 893 207 B1**
(45) Veröffentlichungstag und Bekanntmachung des Hinweises auf die Patenterteilung: **03.08.2011**
(21) Anmeldenummer: 06753700.1
(22) Anmeldetag: 18.05.2006
(51) Int. Cl.: A61K 31/495, A61K 31/496, A61K 31/5375, A61K 31/5377, A61P 17/00, C07D 241/04

(54) **VERWENDUNG VON SUBSTITUIERTEN PIPERAZIN- UND MORPHOLINDERIVATEN**
USE OF SUBSTITUTED PIPERAZINE DERIVATIVES AND MORPHOLINE DERIVATIVES
UTILISATION DE DERIVES DE PIPERAZINE ET DE MORPHOLINE SUBSTITUES

(30) Priorität: 16.06.2005 DE 102005027865; 22.12.2005 DE 102005061657
(43) Veröffentlichungstag der Anmeldung: 05.03.2008
(73) Patentinhaber: Merck Patent GmbH, 64293 Darmstadt (DE)
(72) Erfinder: MUJICA-FERNAUD, Teresa, 64289 Darmstadt (DE); BUCHHOLZ, Herwig, 60599 Frankfurt (DE)
(86) Internationale Anmeldenummer: PCT/EP2006/004709
(87) Internationale Veröffentlichungsnummer: WO 2006/133784

(56) Entgegenhaltungen:
- EP-A- 1 300 402
- WO-A-01/54679
- WO-A-02/072031
- WO-A-03/014121
- WO-A-2004/014387
- WO-A-2005/047249
- US-A1- 2003 202 950
- US-B1- 6 172 229

## Beschreibung

Gegenstand der Erfindung ist die Verwendung von substituierten Piperazin- und Morpholinderivaten zur Prophylaxe und/oder Behandlung von Pigmentstörungen wie Hyperpigmentierung (Chloasma), Sommersprossen (Epheliden), Altersflecken (Lentigines), Sonnenflecken (Lentigo solaris) und umweltbedingter Hautalterung sowie neue substituierte Piperazinderivate.

Ferner betrifft die Erfindung Zubereitungen mit einem wirksamen Gehalt an Piperazin- und Morpholinderivaten. Insbesondere betrifft die vorliegende Erfindung kosmetische Zubereitungen zur Prophylaxe und/oder Behandlung von Pigmentstörungen wie Hyperpigmentierung. Sommersprossen, Altersflecken, post-inflammatorische Pigmentierungen (nach entzündlichen Prozessen) sowie auch umweltbedingter Hautalterung.

90% aller 50jährigen Frauen leiden an Pigmentflecken, aber die unschönen Tupfer, die sich vorwiegend an Dekolle14 und Händen zeigen, sind auch bei 40jährigen keine Seltenheit. Altersflecken entwickeln sich aber auch bei Frauen, die regelmäßig die Pille einnehmen, während der Schwangerschaft oder durch UV-Licht. Sonnen- und Altersflecken treten bei Frauen häufiger auf wie bei Männern. Altersflecken sind völlig harmlos und zeigen auch nach Jahren keine Tendenz zur bösartigen Umwandlung.

Haut- und Haarfarbe sind abhängig vom Gehalt, Größe und Typ des Melanins (einem stickstoffhaltigen dunklen Farbstoff) welches aus Melanozyten, den zur Melaninbildung befähigten Zellen, produziert wird. Ausgehend von Tyrosin und der Hilfe von verschiedenen Melanozyten spezifischen Enzymen wie Tyrosinase oder Tyrosinase-verwandten Proteinen, wird Melanin innerhalb der Melanosomen produziert, mit anschließender Umwandlung der Melanosomen in Keratinozyten. Obwohl das Melanin in der Haut ein geeigneter Schutz gegen UV-Strahlung ist, kann dunklere oder überpigmentierte Haut, wie schon erwähnt, die Schönheit beeinflussen und zu ernsthaften ästhetischen Problemen führen. Hyperpigmentierte Hautbedingungen oder Läsionen enthalten Melasma (auch Chloasma genannt), d.h. unregelmäßig gestaltete gelblich-braune Flecken.

Generell unterscheidet man bei Pigmentflecken zwischen Sommersprossen (Epheliden), Altersflecken (Lentigines), sogenannten Alterswarzen (Verrucae seborrhoicea) und einer Hyperpigmentierung (z.B. Chloasma oder Melasma) und sehr häufig spielt die Sonne hier eine wichtige Rolle. Zu Sommersprossen neigen vor allem Menschen mit sehr heller Haut und rötlichen Haaren. Hyperpigmentierung (Chloasma) findet man hingegen häufig bei jenen Frauen, die regelmäßig ihrem Körper Östrogene zuführen.

Vorbeugen kann man vor allem durch regelmäßigen Sonnenschutz mit einem hohen Lichtschutzfaktor. Ist es aber einmal passiert, so bieten sich verschiedene Möglichkeiten wie Laser, Dermabrasio oder andere elektrochirurgische Verfahren sowie sogenannte Bleichcremes an, um die unschönen Altersflecken zu entfernen. Letztere Alternative (Bleichcremes) hat den Vorteil, dass sie für den Patienten wesentlich kostengünstiger als die elektrochirurgischen Verfahren ist.

Daher war es Aufgabe der vorliegenden Erfindung neue Verbindungen herzustellen, die die Fähigkeit zur Hautaufhellung besitzen.

Eine große Zahl von Verbindungen mit hautaufhellender Wirkung zur Behandlung von Pigmentflecken ist auf dem Markt verfügbar.

Unter anderem sind dies Verbindungen wie Hydrochinon (ist als Wirkstoff in der Kosmetik nicht mehr erlaubt), Kojicsäure, Arbutin, Aloesin oder Rucinol, die die Melaminproduktion in der Haut unterbinden. Sie verzögern die Umwandlung von Tyrosin in Melanin durch Blockade des Enzyms Tyrosinase.

Diese Verbindungen haben jedoch eine Reihe von Nachteilen, wie z.B. geringe Depigmentierungs-Effizienz, Nebenwirkungen wie Hautirritationen oder Hautexfoliation (Hautabschälung), Zellschädigungen, geringe Hautdurchdringung oder geringe Haltbarkeit der Formulierungen. Daher ist ein Bedürfnis nach neuen Hautaufhellern mit höherer Effektivität vorhanden.

Überraschenderweise ist nun gefunden worden, dass bestimmte substituierte Piperazin- und Morpholinderivate der Ia bis IIp exzellente Hautaufheller-Eigenschaften besitzen. Sie unterbinden die Synthese von Melanin, verhindern die Melanin-Überproduktion und sind somit zur Behandlung von Pigmentflecken aller Art geeignet,

Gegenstand der vorliegenden Erfindung ist somit die nicht-medizinische Verwendung von Verbindungen der Formeln Ia bis IIp oder ihrer physiologisch unbedenklichen Salze

| Strukturformel | |
|---|---|
| 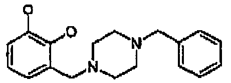 | **Ia** |
| 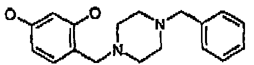 | **Ib** |
| 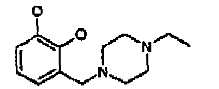 | **Ic** |
| 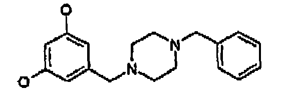 | **Id** |
| 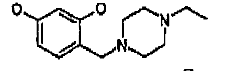 | **Ie** |
| 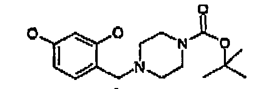 | **If** |
| 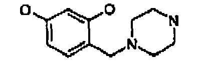 | **Ig** |
| 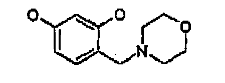 | **Ih** |
| 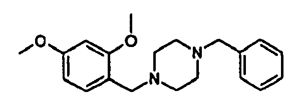 | **Ii** |
| 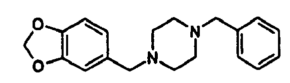 | **Ij** |
| 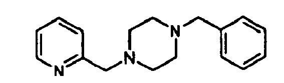 | **IIk** |
| 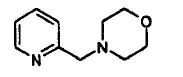 | **IIm** |
| 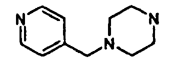 | **IIn** |
| 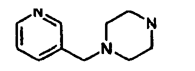 | **IIo** |
| 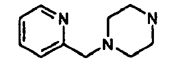 | **IIp** |

oder einer Zubereitung enthaltend mindestens eine Verbindung der Formeln Ia bis IIp, zur Prophylaxe und/oder Behandlung von Pigmentstörungen wie Hyperpigmentierung, Sommersprossen, Altersflecken sowie umweltbedingter Hautalterung.

Grundsätzlich sind im Sinne der vorliegenden Erfindung von der Bezeichnung "Verbindung nach den Formeln Ia bis IIp" auch die Salze der Verbindungen nach Formel Ia bis IIp umfasst. Zu den bevorzugten Salzen gehören dabei insbesondere Alkali- und Erdalkalimetallsalze sowie Ammonium-Salze, insbesondere jedoch Natrium- und Kalium-Salze.

Ein weiterer Gegenstand der vorliegenden Erfindung sind substituierte Piperazinderivate mit den Formeln Ia bis Ie:

| | |
|---|---|
| 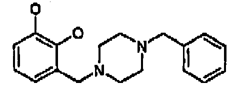 | Ia |
| 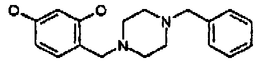 | Ib |
| 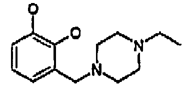 | Ic |
| 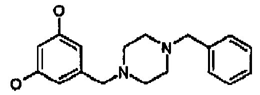 | Id |
| 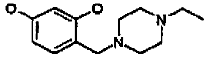 | Ie |

Ein weiterer Gegenstand der vorliegenden Erfindung sind Zubereitungen enthaltend mindestens eine Verbindung nach den Formeln Ia bis IIp gemäß Anspruch 1 sowie mindestens einen weiteren hautpflegenden Inhaltsstoff und mindestens einen für topische Anwendungen geeigneten Träger.

Anwendungen strukturell verwandter Verbindungen sind aus der Literatur bekannt:
Aus DE 210 10 356 U1 sind m-Dihydroxybenzol-Derivate bekannt, die zum oxidativen Färben von Keratinfasern verwendet werden. Aus WO 03/014121 A1 sind PARP (poly(ADP-ribose)polymerase)- Inhibitoren bekannt, die gegen Hautalterung eingesetzt werden können.

Die Verbindungen der Formeln Ia bis IIp werden erfindungsgemäß üblicherweise in Mengen von 0.01 bis 20 Gew.%, vorzugsweise in Mengen von 0.1 bis 10 Gew.% eingesetzt. Dabei bereitet es dem Fachmann keinerlei Schwierigkeiten die Mengen abhängig von der beabsichtigten Wirkung der Zubereitung entsprechend auszuwählen.

Die schützende Wirkung gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet, geeignet schnell oder zeitverzögert wirkende Antioxidantien auszuwählen.

In einer bevorzugten Ausführungsform der vorliegenden Erfindung handelt es sich bei mindestens seinem weiteren hautpflegenden Inhaltsstoff um ein oder mehrere Antioxidantien und/oder Vitamine.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z:B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbyläcetat), Tocopherole und Derivate (z.B. Vitamin-E-acetat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit Verbindungen der Formeln I und II in solchen Zusammensetzungen überlicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Zubereitungen enthalten, insbesondere bevorzugt Vitamin C und dessen Derivaten, DL-α-Tocopherol, Tocopherol-E-Acetat, Nicotinsäure, Pantothensäure und Biotin. Vitamine werden dabei mit Verbindungen der Formel I überlicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind weiter Verbindungen der Formel V 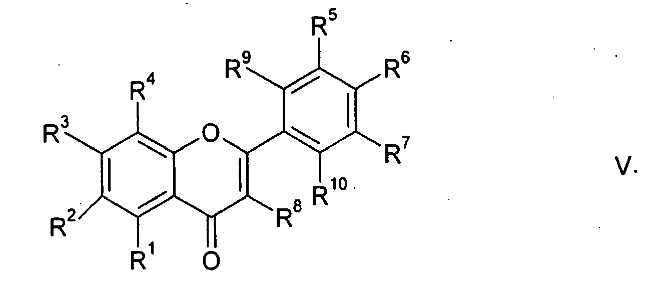 wobei R¹ bis R¹⁰ gleich oder verschieden sein können und ausgewählt sind aus
- H
- OR"
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
- wobei alle OR¹¹ unabhängig voneinander stehen für
   - OH
   - geradkettige oder verzweigte C₁- bis C₂₀-Alkyloxygruppen,
   - geradkettigen oder verzweigten C₃- bis C₂₀-Alkenyloxygruppen,
   - geradkettigen oder verzweigten C₁- bis C₂₀-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
   - C₃- bis C₁₀-Cycloalkyloxygruppen und/oder C₃- bis C₁₂-Cycloalkenyloxygruppen, wobei die Ringe jeweils auch durch -(CH₂)ₙ-Gruppen mit n = 1 bis 3 überbrückt sein können und/oder,
   - Mono- und/oder Oligoglycosylreste,
      mit der Maßgabe, dass mindestens 4 Reste aus R¹ bis R⁷ stehen für OH und dass im Molekül mindestens 2 Paare benachbarter Gruppen -OH vorliegen,

- oder R², R⁵ und R⁶ für OH und die Reste R¹, R³, R⁴ und R⁷⁻¹⁰ für H stehen,
   wie sie in der älteren Deutschen Patentanmeldung DE 10244282.7 beschrieben sind.

Vorteile der erfindungsgemäßen Zusammensetzungen enthaltend mindestens ein Antioxidans sind dabei neben den oben genannten Vorteilen insbesondere die antioxidante Wirkung und die gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der Verbindungen nach Formel V, welches sich im DPPH-Assay in einer hohen Kapazität Radikale zu fangen (EC₅₀), einer zeitverzögerten Wirkung (T_{EC50} > 120 min) und damit einer mittleren bis hohen antiradikalischen Effizienz (AE) äußert. Zudem vereinigen die Verbindungen nach Formel V im Molekül antioxidative Eigenschaften mit UV-Absorption im UV-A- und/oder -B-Bereich. Bevorzugt sind daher auch Zubereitungen enthaltend zumindest eine Verbindung der Formel V, die dadurch gekennzeichnet ist, dass mindestens zwei benachbarte Reste der Reste R¹ bis R⁴ stehen für OH und mindestens zwei benachbarte Reste der Reste R⁵ bis R⁷ stehen für OH. Insbesondere bevorzugte Zubereitungen enthalten zumindest eine Verbindung der Formel V, die dadurch gekennzeichnet ist, dass mindestens drei benachbarte Reste der Reste R¹ bis R⁴ stehen für OH, wobei vorzugsweise die Reste R¹ bis R³ für OH stehen.

Damit die Verbindungen der Formeln Ia bis IIp ihre positive Wirkung auf die Haut besonders gut entwickeln können, kann es bevorzugt sein die Verbindungen der Formeln Ia bis IIp in tiefere Hautschichten eindringen zu lassen. Dazu stehen mehrere Möglichkeiten zur Verfügung. Zum einen können die Verbindungen der Formeln Ia bis IIp ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formeln Ia bis IIp durch die äußeren Hautschichten ermöglichen, Schließlich ist auch ein systemischer Transport der Verbindungen der Formel Ia bis Ij denkbar. Die Zubereitung wird dann beispielsweise so gestaltet, dass sie für eine orale Gabe geeignet ist.

Insbesondere eignen sich die erfindungsgemäßen Zusammensetzungen auch zur Behandlung von Hautkrankheiten, die durch UV-Strahlung hervorgerufen werden.

Erfindungsgemäß bevorzugte Zubereitungen enthalten neben den Verbindungen der Formel Ia bis Ij auch UV-Filter.

Bei Einsatz der als UV-A-Filter insbesondere bevorzugten Dibenzoylmethanderivate in Kombination mit den Verbindungen der Formeln Ia bis IIp ergibt sich ein zusätzlicher Vorteil: Die UV-empfindlichen Dibenzoylmethanderivate werden durch die Anwesenheit der Verbindungen der Formeln Ia bis IIp zusätzlich stabilisiert. Ein weiterer Gegenstand der vorliegenden Erfindung ist daher die Verwendung der Verbindungen gemäß Formeln Ia bis IIp zur Stabilisierung von Dibenzoylmethanderivaten in Zubereitungen.

Prinzipiell kommen alle UV-Filter für eine Kombination mit den erfindungsgemäßen Verbindungen der Formeln Ia bis IIp in Frage.

Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Memoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl®. SK) oder (2-Oxoborn-3-yliden)toluol-4-sulfonsäure (z.B. Mexoryl® SL),
Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),
Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
Methoxyzimtsäureester wie Methoxyzimtsäureoctylester (z. B. Eusolex® 2292), 4-Methoxyzimtsäureisopentylester, z.B. als Gemisch der Isomere (z.B. Neo Heliopan® E 1000),
Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),
4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),
Phenylbenzimidazolsulfonsäuren, wie 2-Phenylbenzimidazol-5-sulfonsäure sowie ihre Kalium-, Natrium- und Triethanolaminsalze (z.B.. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.
-2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
-4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),

Weitere geeignete UV-Filter sind auch Methoxyflavone entsprechend der älteren Deutschen Patentanmeldung DE 10232595.2.

Organische UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex^{®}T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy-benzophenon, Methoxyzimtsäureoctylester, 3,3,5-Trimethyl-cyclo-hexylsalicylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanolaminsalze.

Durch Kombination von einer oder mehrerer Verbindungen der Formeln Ia bis IIp mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgenden Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, welche die Photostabilität dieser Filter beeinträchtigen, wie beispielsweise Zimtsäurederivate, kann die Photostabilität der gesamten Zubereitung erhöht werden.
- in der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben. Geeignete Kapseln können Wände aus anorganischen oder organischen Polymeren aufweisen. Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Alle Verbindungen oder Komponenten, die in den Zubereitungen verwendet werden können, sind entweder bekannt und käuflich erwerbbar oder können nach bekannten Verfahren synthetisiert werden. Die Herstellung der neuen Verbindungen nach den Formeln Ia bis IIp weiter unten beschrieben.

Eine oder mehrere substituierte Piperazin- und Morpholinderivate der Formeln Ia bis IIp können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Öle, Aerosole, Sprays oder auch Sticks. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z.B. in Kollagenmatrices und anderen üblichen Verkapselungsmaterialien.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formeln Ia bis IIp mit Resten wie oben beschrieben mit einem kosmetisch oder dermatologisch geeigneten Träger vermischt wird, und die Verwendung einer Verbindung der Formeln Ia bis IIp zur Herstellung einer Zubereitung.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß den Formeln Ia bis IIp in dem Träger zur Folge haben.

In einem erfindungsgemäß bevorzugten Verfahren werden die Verbindungen gemäß Formel Ia bis Ij hergestellt durch die Reaktion eines entsprechend substituierten Aldehyds der Formel III (siehe Schema 1) mit einem Amin der Formel IV in Gegenwart eines Reduktionsmittels (siehe J.Org. Chem. 1996,61, 849).

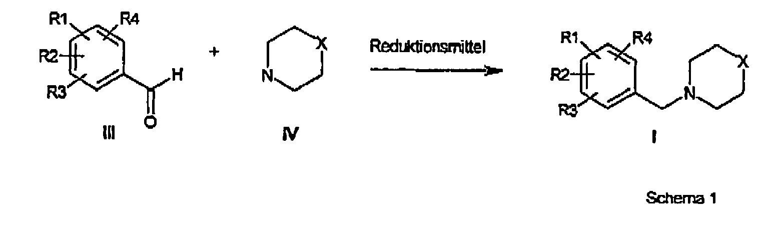

Analog dazu werden gemäß Schema 2 Verbindungen nach der Formel IIk bis IIp durch reduktive Aminierung eines Pyrdin-Carbaldehyds V mit einem Amin IV hergestellt.

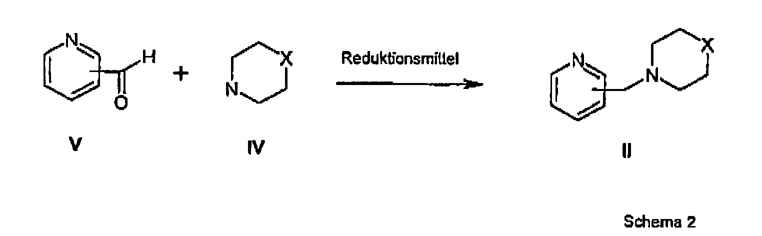

Durch ihre Wirkung eignen sich Verbindungen der Formeln Ia bis IIp auch als Arzneimittelwirkstoffe.

Die folgenden Beispiele sollen die vorliegende Erfindung näher erläutern ohne sie einzuschränken.

### Beispiel 1:

### Herstellung von 3-(4-Benzyl-piperazin-1-ylmethyl)-benzen-1,2-diol (Ia)

Zu 2.17 mmol (300mg) 2,3-Dihydoxybenzaldehyd IIIa und 2.39 mmol (0.42 ml) N-Benzylpiperazin in 1,2-Dichlormethan (20 ml) werden bei Raumtemperatur unter Schutzgas 3.26 mmol (690 mg) Natriumtriacetoxyborhydrid sowie 2 Tr. Essigsäure zugegeben und das Reaktionsgemisch 24 h gerührt. Danach wird das Reaktionsgemisch mit kalter wässriger Lösung abgeschreckt und über Wasser (30 ml) und Dichlormethan (30 ml) aufgeteilt Die wässrige Phase wird mit gesättigter NaHCO₃-Lösung auf pH=9 gebracht und mit zweimal 30 ml Dichlormethan extrahiert. Anschließend werden die vereinigten Phasen über MgSO₄ getrocknet und das Lösungsmittel im Vakuum abgezogen. Der Feststoff wird vorsichtig aus Ethanol umkristallisiert, sodass die Verbindung Ia entsteht.

### Charakterisierung:

¹H NMR (DMSO-d6) δ 7.36-7.20 (m, 5H), 6.65 (dd, *J*= 7.4, 1.9 Hz, 1H), 6.55 (t, *J*= 7.4 Hz, 1H), 6.51-6.47 (m, 1H), 3.61 (s, 2H), 3.48 (s, 2H), 2.48-2.35 (m, 8H).
EI MS *m*/*z*: 298 (M+). 207, 175, 91

### Beispiel 2:

### - Hemmung des Enzyms Tyrosinase

Die Wirkung der Verbindungen nach Formeln Ia bis IIp als Hautaufheller wird geprüft durch ihre Fähigkeit das Enzym Tyrosinase zu hemmen und damit die Melanin-Synthese zu unterbinden. Um die hemmende Wirkung der Verbindungen der Formeln Ia bis IIp gegenüber Tyrosinase zu zeigen wurden als Substrate Pilz-tyrosinase und L-Tyrosin oder L-DOPA verwendet und mit der Referenz-Verbindung verglichen.

### - Tyrosinase-Versuch

Die Verbindungen Ia bis IIp und Tyrosinase (10 U) werden 10 Min. in Eis vorgekühlt und L-DOPA (4 mM) zugefügt und für 1 h auf 37°C gehalten. Es wird die optische Dichte jedes experimentell ermittelten Punktes bei 450 nm gegenüber der entsprechenden Referenz ohne Enzym gemessen. Kojicsäure wird als Tyrosinase-Referenz-Inhibitor benutzt. Die Versuchsergebnisse sind in der folgenden Tabelle 1 dargestellt, ausgedrückt durch den IC 50-Wert (d.h. die Konzentration der jeweiligen Versuchssubstanz bei der die Tyrosinase-Aktivität um 50% blockiert ist).

**Tab. 1 Tyrosinase-Versuchsergebnisse**

| **Verbindung** | **IC₅₀ (µM)** |
|---|---|
| **Kojicsäure** | 12 |
| **Ia** | 134 |
| **Ib** | 154 |
| **Id** | >1mM |
| **Ie** | 263 |
| **If** | 140 |
| **Ih** | 78 |
| **Ii** | >1mM |
| **Ij** | 153 |
| **IIk** | >1mM |
| **IIn** | 280 |
| **IIo** | 360 |
| **IIp** | 553 |

### Beispiel3:

### Untersuchung des Effektes der Melaninsynthese an menschlichen Melanocyten-Kulturen (Melanin Versuch)

Das Potential der Verbindungen nach Formeln Ia bis IIp als Hautaufheller-Wirkstoff wurde außerdem durch den Melanin-Versuch verifiziert. Der Effekt in Bezug auf den Melanin-Gehalt von menschlichen epidermalen Melanocyten-Kulturen dieser Verbindungen wurde ermittelt und mit der Referenzverbindung (Kojicsäure) verglichen.

### Melanin-Versuch:

Eine vorausgehende Cytotoxizitäts-Studie der Verbindungen nach Formeln Ia bis IIp wurde durchgeführt um drei nicht-cytotoxische Konzentrationen für den Melanin-Versuch zu ermitteln.

Melanin-Versuch: normale menschliche Melanocyten wurden in 24 Lochplatten (110.000 Zellen/Loch) kultiviert. Bei 50 % Zusammenfluss wurde das Kulturmedium mit oder ohne (Kontrollverbindung) Verbindungen der Formeln Ia bis IIp durch frisches Kulturmedium in drei verschiedenen Konzentrationen ersetzt.

Nach der Inkubation (240 Stunden) wurden die Monolayers gewaschen, die Zellen lysiert und das Melanin extrahiert. Es wurde die optische Dichte jedes experimentellen Punktes bei 405 nm gegen den Melanin-Standard gemessen. Tabelle 2 zeigt die Ergebnisse zwei ausgewählter Verbindungen im Vergleich zur Kojicsäure und der Kontrollverbindung (mit 100% Melaningehalt), wobei der ermittelte Melaningehalt (in %) bei jeweils drei verschiedenen Konzentrationen angegeben ist.

**Tab. 2 Melanin-Versuchsergebnisse**

| **Verbindung** | **Konzent.** (µg/mL) | **Melanin** (µg/mL) | **% Kontrolle** | **Proteine (mg/mL)** | **Melanin normiert*** (µglmL) | **% Kontrolle normiert*** |
|---|---|---|---|---|---|---|
| **Kontroll-Verbindung** | - | 37.2 | 100 | 0.477 | 37.2 | 100 |
| **Kojicsäure** | 36 | 23.8 | 64 | 0.358 | 31.7 | 85 |
| | 15.6 | 30 | 80 | 0.431 | 34.6 | 92 |
| | 6.8 | 34.4 | 92 | 0.450 | 38.0 | 101 |
| **Ia** | 0.8 | 38.6 | 104 | 0.504 | 36.4 | 98 |
| **Ib** | 4 | 37.1 | 100 | 0.508 | 34.8 | 94 |
| | 0.8 | 37.8 | 102 | 0.486 | 37.1 | 100 |
| **Ie** | 20 | 33.8 | 91 | 0.490 | 32.9 | 88 |
| | 4 | 37.5 | 101 | 0.497 | 36.0 | 97 |
| | 0.8 | 37.3 | 100 | 0.488 | 36.5 | 98 |
| **Ih** | 40 | 30.6 | 82 | 0.48 | 30.4 | 82 |
| | 8 | 35.3 | 95 | 0.525 | 32.1 | 86 |
| | 1.6 | 37.2 | 100 | 0.48 | 37.0 | 99 |

### Beispiel 4:

### Herstellung von 1-Benzyl-4-pyridin-2-ylmethyl-piperazin (IIk)

Zu 2.61 mmol (0.25ml) Pyridin-2-carbaldehyd IIIk und 2.88 mmol (0.51 ml) N-Benzylpiperazin in 1,2-Dichlormethan (25 ml) werden bei Raumtemperatur unter Schutzgas 3.92 mmol (830 mg) Natriumtriacetoxyborhydrid sowie 2 Tr. Essigsäure zugegeben und das Reaktionsgemisch 24 h gerührt. Danach wird das Reaktionsgemisch mit kalter wässriger Lösung abgeschreckt und über Wasser (30 ml) und Dichlormethan (30 ml) aufgeteilt. Die wässrige Phase wird mit gesättigter NaHCO₃-Lösun auf pH=9 gebracht und mit zweimal 30 ml Dichlormethan exrahiert. Anschließend werden die vereinigten Phasen über MgSO₄ getrocknet und das Lösungsmittel im Vakuum abgezogen. Der Feststoff wird vorsichtig aus Ethanol umkristallisiert, sodass die Verbindung IIk entsteht.

### Beispiel 5:

### Herstellung von 4-(4-Benzyl-piperazin-1-ylmethyl)-benzen-1,3-diol (Ib)

Zu einer Lösung von 7,24 mmol (1g) 2,4-Dihydroxybenzaldehyd IIIb und 7.97 mmol (1.4 ml) Benzylpiperazin in 50 ml 1,2 Dichlormethan werden bei Raumtemperatur unter Schutzgas 10.86 mmol Natriumtriacetoxyborhydrid und 3 Tr. Essigsäure zugegeben und das Reaktionsgemisch 24 h bei Raumtemperatur gerührt. Danach wird das Reaktionsgemisch mit kalter wässriger Lösung abgeschreckt und über Wasser (50 ml) und Dichlormethan (50 ml) aufgeteilt. Die vereinigten wässrigen Phasen werden durch Zugabe von gesättigter NaHCO₃-Lösung alkalisch (pH 9) gemacht und zweimal mit Dichlormethan extrahiert. Anschließend werden die vereinigten Phasen über MgSO₄ getrocknet und das Lösungsmittel im Vakuum abgezogen. Der Feststoff wird vorsichtig aus Ethanol umkristallisiert, sodass die Verbindung Ib ensteht.

### Charakterisierung:

¹H NMR (DMSO-d6) δ 7.36-7.20 (m, 5H), 6.80 (d, *J*= 8.7 Hz, 1H), 6.45 (m, 2H), 3.50 (s, 2H), 3.47 (s, 2H), 3.37-3.30 (m, 4H), 2.48-2.33 (m, 4H).
EI MS *m*/*z*: 298 (M+), 207, 175, 91

### Beispiel 6:

### Herstellung von 3-(4-Ethyl-piperazin-1-ylmethyl)-benzen-1,2-diol (Ic)

Zu einer Lösung von 2.17 mmol (300 mg) 2,3-Dihydroxybenzaldehyd IIIa und 2.39 mmol (0.31 ml) Ethylpiperazin in Dichlormethan werden bei Raumtemperatur unter Schutzgas 3.26 mmol (690 mg) Natriumtriacetoxyborhydrid und 2 Tr. Essigsäure zugegeben und das Reaktionsgemisch 24 h bei Raumtemperatur gerührt. Danach wird das Reaktionsgemisch mit kalter wässriger Lösung abgeschreckt und über Wasser (30 ml) und Dichlormethan (30 ml) aufgeteilt. Die vereinigten wässrigen Phasen werden durch Zugabe von gesättigter NaHCO₃-Lösung alkalisch (pH 9) gemacht und zweimal mit Dichlormethan extrahiert. Anschließend werden die vereinigten Phasen über MgSO₄ getrocknet und das Lösungsmittel im Vakuum abgezogen. Der Feststoff wird vorsichtig aus Ethanol umkristallisiert, sodass die Verbindung **Ic** entsteht.

### Charakterisierung:

¹H NMR (DMSO-d6) δ 6.66 (dd, *J*= 7.5, 2.0 Hz, 1H), 6.56 (t, J= 7.5 Hz, 1H), 6.51 (dd, J= 7.5, 2.0 Hz, 1H), 3.63 (s, 2H), 3.59-3.30 (m, 8H), 2.33 (q,
J= 7.2 Hz, 2H), 1.00 (t, J= 7.2 Hz, 3H).
APCI MS *m*/*z*: 237 (M+H)⁺

### Beispiel 7:

### Herstellung von 5-(4-Benzyl-piperazin-1-ylmethyl)-benzen-1,3-diol (Id)

Zu einer Lösung von 2.17 mmol (300 mg) 2, 3-Dihydroxybenzaldehyd IIIc und 2.17 mmol (0.47 ml) Benzylpiperazin in Dichlormethan (20 ml) werden bei Raumtemperatur unter Schutzgas 3.26 mmol (690 mg) Natriumtriacetoxyborhydrid und 2 Tr. Essigsäure zugegeben und das Reaktionsgemisch 24 h bei Raumtemperatur gerührt. Danach wird das Reaktionsgemisch mit kalter wässriger Lösung abgeschreckt und über Wasser (30 ml) und Dichlormethan (30 ml) aufgeteilt. Die vereinigten wässrigen Phasen werden durch Zugabe von gesättigter NaHCO₃-Lösung alkalisch (pH 9) gemacht und zweimal mit Dichlormethan extrahiert. Anschließen werden die vereinigten Phasen über MgSO₄ getrocknet und das Lösungsmittel im Vakuum abgezogen. Der Feststoff wird vorsichtig aus Ethanol umkristallisiert, sodass die Verbindung **Id** entsteht.

### Charakterisierung:

¹H NMR (CDCl₃) δ 7.27 (m, 5H), 6.25 (bs, 2H), 6.15 (bs, 1H), 3.49 (s, 2H), 3.36 (s, 2H), 3.13-2.56 (m, 4H), 2.54-2.40 (m, 4H).
APCI MS *m*/*z*: 299 (M+H)⁺

### Beispiel 8:

### Herstellung von 4-(4-Ethyl-piperazin-1-ylmethyl)-benzen-1,3-diol (le)

Zu einer Lösung von 7.24 mmol (1.0 g) 2,3-Dihydroxybenzaldehyd IIIb und 7.97 mmol (1.0 ml) Ethylpiperazin in Dichlormethan werden bei Raumtemperatur unter Schutzgas 10.86 mmol (2.3 g) Natriumtriacetoxyborhydrid und 3 Tr. Essigsäure zugegeben und das Reaktionsgemisch 24 h bei Raumtemperatur gerührt. Danach wird das Reaktionsgemisch mit kalter wässriger Lösung abgeschreckt und über Wasser (50 ml) und Dichlormethan (50 ml) aufgeteilt. Die vereinigten wässrigen Phasen werden durch Zugabe von gesättigter NaHCO₃-Lösung alkalisch (pH 9) gemacht und zweimal mit Dichlormethan extrahiert. Anschließend werden die vereinigten Phasen über MgSO₄ getrocknet und das Lösungsmittel im Vakuum abgezogen. Der Feststoff wird vorsichtig aus Ethanol umkristallisiert, sodass die Verbindung **Ie** entsteht.

### Charakterisierung:

¹H NMR (DMSO-d6) δ 6.80 (dd, *J*= 8.6 Hz, 1H), 6.15 (m, 2H), 3.50 (s, 2H), 3.40-3.16 (m, 4H),2.48- 2.32 (m, 4H), 2.30 (q, J= 7.1 Hz, 2H), 0.98 (t, J= 7.1 Hz, 3H).
EI MS *m*/*z*: 236 (M⁺), 151, 122, 72

### Beispiel 9:

### Herstellung von 4-(4-tert-Butoxycarbinyl-piperazin-1-ylmethyl)-benzen-1,3-diol (If)

Die Herstellung erfolgt analog zu Beispiel 1 gemäß Reaktionsschema 1

### Beispiel 10:

### Herstellung von 4-(Piperazin-1-ylmethyl)-benzen-1,3-diol (Ig)

Die Herstellung erfolgt analog zu Beispiel 1 gemäß Reaktionsschema 1

### Beispiel 11:

### Herstellung von 4-Morpholin-1-ylmethyl-benzen-1,3-diol (Ih)

Zu einer Lösung von 3.62 mmol (500 mg) 2,3-Dihydroxybenzaldehyd IIIb und 3.98 mmol (0.35 ml) Morpholin in Dichlormethan werden bei Raumtemperatur unter Schutzgas 5.43 mmol (1.15 g) Natriumtriacetoxyborhydrid und 2 Tr. Essigsäure zugegeben und das Reaktionsgemisch 24 h bei Raumtemperatur gerührt. Die weitere Aufarbeitung erfolgt analog zu den vorherigen Beispielen.

### Charakterisierung:

¹H NMR (DMSO-d6) δ 6.84 (dd, *J*= 8.1 Hz, 1H), 6.20-6.13 (m, 2H), 3.57 (t, J= 4.5 Hz, 4H), 3.45 (s, 2H), 3.43-3.25 (m, 2H), 2.39 (m, 2H) EI MS *m*/*z*: 209 (M⁺), 123, 86

### Beispiel 12:

### Herstellung von 1-Benzyl-4-(2,4-dimethoxy-benzyl)-piperazin (Ii)

Zu einer Lösung von 6.02 mmol (1.0 g) 2,3-Dimethoxybenzaldehyd IIId und 6.62 mmol (1.16 ml) Benzylpiperazin in Dichlormethan werden bei Raumtemperatur unter Schutzgas 9.03 mmol (1.91 g) Natriumtriacetoxyborhydrid und 3 Tr. Essigsäure zugegeben und das Reaktionsgemisch 24 h bei Raumtemperatur gerührt. Danach wird das Reaktionsgemisch mit kalter wässriger Lösung abgeschreckt und über Wasser (50 ml) und Ethylacetat (50 ml) aufgeteilt. Die vereinigten wässrigen Phasen werden mit Ethylacetat extrahiert. Anschließend werden die vereinigten Phasen über MgSO₄ getrocknet und das Lösungsmittel im Vakuum abgezogen. Der Feststoff wird vorsichtig aus MTBE umkristallisiert, sodass die Verbindung Ii entsteht.

### Charakterisierung:

EI MS *m*/*z*: 326 (M⁺), 235, 175, 151, 91

### Beispiel 13:

### Herstellung von 1-Benzo[1,3]dioxol-5-ylmethyl-4-benzyl-piperazin (Ij)

Zu einer Lösung von 6.66 mmol (1.0 g) Benzo[1,3]dioxole-5-carbaldehyd **IIIe** und 7.33 mmol (1.28 ml) Benzylpiperazin in Dichlormethan werden bei Raumtemperatur unter Schutzgas 9.99 mmol (1.12 g) Natriumtriacetoxyborhydrid und 3 Tr. Essigsäure zugegeben und das Reaktionsgemisch 24 h bei Raumtemperatur gerührt. Danach wird das Reaktionsgemisch mit kalter wässriger Lösung abgeschreckt und über Wasser (50 ml) und Ethylacetat (50 ml) aufgeteilt. Die vereinigten wässrigen Phasen werden durch Zugabe von Ethylacetat (2x 50 ml) extrahiert. Anschließend werden die vereinigten Phasen über MgSO₄ getrocknet und das Lösungsmittel im Vakuum abgezogen. Der Feststoff wird vorsichtig aus MTBE umkristallisiert, sodass die Verbindung **Ij** entsteht.

### Charakterisierung:

EI MS *m*/*z*: 310 (M⁺), 219, 175, 135, 91.

### Beispiel 14:

### Herstellung von 4-Pyridin-2-ylmethyl-morpholin (IIm)

Die Herstellung erfolgt analog zu Beispiel 3 gemäß Reaktionsschema 2

### Beispiel 15:

### Herstellung von 1-Pydridin-4-ylmethyl-piperazin (IIn)

Die Herstellung erfolgt analog zu Beispiel 3 gemäß Reaktionsschema 2

### Beispiel 16:

### Herstellung von 1-Pyridin-3-ylmethyl-piperazin (IIo)

Die Herstellung erfolgt analog zu Beispiel 3 gemäß Reaktionsschema 2

### Beispiel 17:

### Herstellung von 1-Pyridin-2-ylmethyl-piperazin (IIp)

Die Herstellung erfolgt analog zu Beispiel 3 gemäß Reaktionsschema 2

## Patentansprüche

1. Nicht-medizinische Verwendung von substituierten Piperazin- und Morpholinderivaten ausgewählt aus den Verbindungen der Formeln Ia bis IIp oder deren physiologisch unbedenklichen Salze
| Strukturformel | |
|---|---|
| 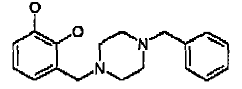 | **Ia** |
| 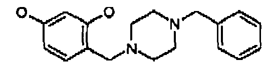 | **Ib** |
| 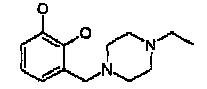 | **Ic** |
| 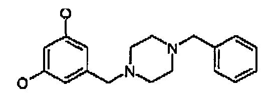 | **Id** |
| 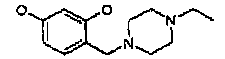 | **Ie** |
| 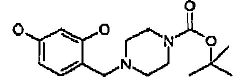 | **If** |
| 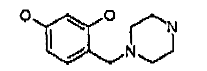 | **Ig** |
| 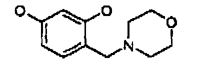 | **Ih** |
| 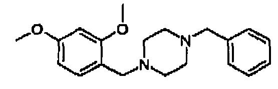 | **Ii** |
| 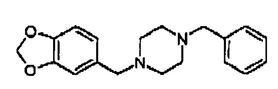 | **Ij** |
| 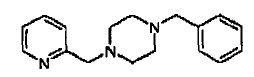 | **IIk** |
| 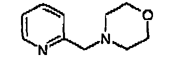 | **IIm** |
| 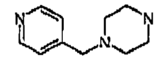 | **IIn** |
| 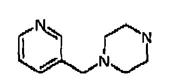 | **IIo** |
| 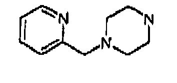 | **IIp** |
oder einer Zubereitung enthaltend mindestens eine Verbindung der Formeln Ia bis IIp zur Prophylaxe und/oder Behandlung von Pigmentstörungen wie Hyperpigmentierung, Sommersprossen, Altersflecken, Sonnenflecken sowie umweltbedingter Hautalterung.

2. Zubereitung, enthaltend mindestens eines substituierten Piperazin- und Morpholinderivates nach den Formeln Ia bis IIp gemäß Anspruch 1, sowie mind31.8estens einen weiteren hautpflegenden Inhaltsstoff und mindestens einen für topische Anwendungen geeigneten Träger.

3. Zubereitung nach Anspruch 2 enthaltend zumindest ein substituiertes Piperazin- und Morpholinderivat der Formeln Ia bis IIp, **dadurch gekennzeichnet, dass** die Zubereitungen eine oder mehrere Verbindungen der Formel Ia bis IIp in einer Menge von 0.01 bis 20 Gew.%, vorzugsweise in einer Menge von 0.1 bis 10 Gew.%, bezogen auf das Gesamtgewicht der Zubereitung, enthalten.

4. Zubereitung nach Anspruch 2 oder 3, **dadurch gekennzeichnet, dass** es sich bei mindestens einem weiteren hautpflegenden Inhaltsstoff um eine oder mehrere Antioxidantien und/oder Vitamine handelt.

5. Zubereitung nach einem oder mehreren der Ansprüche 2 bis 4, wobei die Zubereitung einen oder mehrere UV-Filter enthält, die vorzugsweise ausgewählt sind aus der Gruppe, die 3-(4'-Methyl-benzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze enthält.

6. Verfahren zur Herstellung einer Zubereitung nach Anspruch 2, **dadurch gekennzeichnet, dass** ein substituiertes Piperazin- und Morpholinderivat der Formeln Ia bis IIp nach Anspruch 1 mit einem kosmetisch oder dermatologisch geeignetem Träger vermischt werden.

7. Substituiertes Piperazinderivat ausgewählt aus den Verbindungen mit den Formeln Ia bis Ie
| | |
|---|---|
| 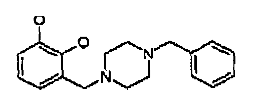 | **Ia** |
| 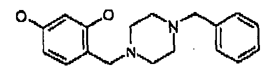 | **Ib** |
| 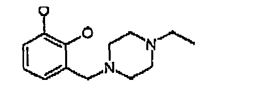 | **Ic** |
| 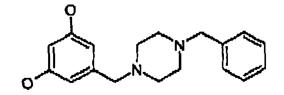 | **Id** |
| 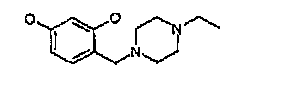 | **Ie** |

## Claims

1. Non-medical use of substituted piperazine and morpholine derivatives selected from the compounds of the formulae Ia to IIp or physiologically acceptable salts thereof
| Structural formula | |
|---|---|
| 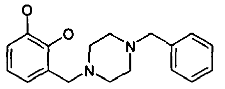 | **Ia** |
| 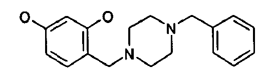 | **Ib** |
| 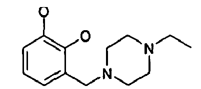 | **Ic** |
| 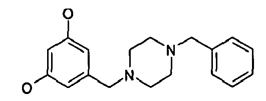 | **Id** |
| 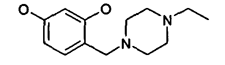 | **Ie** |
| 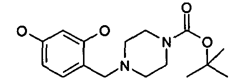 | **If** |
| 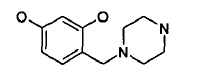 | **Ig** |
| 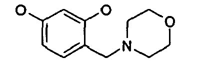 | **Ih** |
| 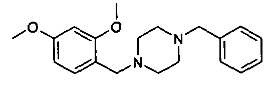 | **Ii** |
| 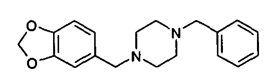 | **Ij** |
| 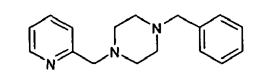 | **IIk** |
| 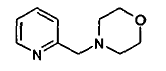 | **IIm** |
| 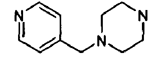 | **IIn** |
| 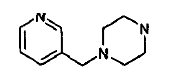 | **IIo** |
| 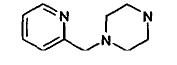 | **IIp** |
or of a composition comprising at least one compound of the formulae la to IIp
for the prophylaxis and/or treatment of pigment defects, such as hyperpigmentation, freckles, age spots, sun spots and environmentally induced skin ageing.

2. Composition comprising at least one substituted piperazine or morpholine derivative of the formulae Ia to IIp according to Claim 1 and at least one further skin-care ingredient and at least one vehicle which is suitable for topical applications.

3. Composition according to Claim 2 comprising at least one substituted piperazine or morpholine derivative of the formulae Ia to IIp, **characterised in that** the compositions comprise one or more compounds of the formulae I and IIp in an amount of 0.01 to 20% by weight, preferably in an amount of 0.1 to 10% by weight, based on the total weight of the composition.

4. Composition according to Claim 2 or 3, **characterised in that** at least one further skin-care ingredient is one or more antioxidants and/or vitamins.

5. Composition according to one or more of Claims 2 to 4, where the composition comprises one or more UV filters, which are preferably selected from the group comprising 3-(4'-methylbenzylidene)-dl-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenyl-acrylate, 2-phenylbenzimidazole-5-sulfonic acid and potassium, sodium and triethanolamine salts thereof.

6. Process for the preparation of a composition according to Claim 2, **characterised in that** a substituted piperazine or morpholine derivative of the formulae Ia to IIp according to Claim 1 is mixed with a cosmetically or dermatologically suitable vehicle.

7. Substituted piperazine derivative selected from the compounds of the formulae Ia to 1e
| | |
|---|---|
| 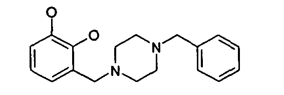 | **Ia** |
| 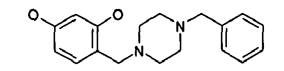 | **Ib** |
| 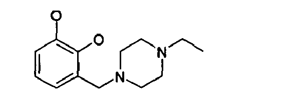 | **Ic** |
| 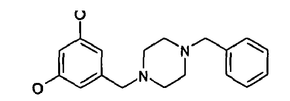 | **Id** |
| 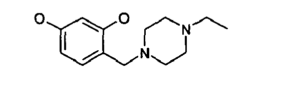 | **Ie** |

## Revendications

1. Utilisation non médicale de dérivés de pipérazine et de morpholine substitués choisis parmi les composés de formules Ia à IIp ou de sels physiologiquement acceptables de ceux-ci
| Formule structurelle | |
|---|---|
| 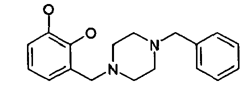 | **Ia** |
| 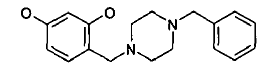 | **Ib** |
| 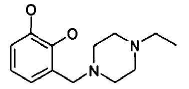 | **Ic** |
| 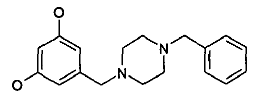 | **Id** |
| 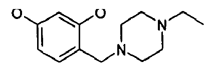 | **Ie** |
| 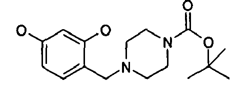 | **If** |
| 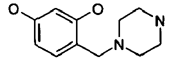 | **Ig** |
| 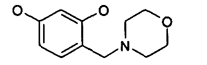 | **Ih** |
| 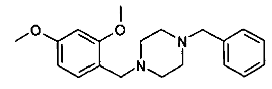 | **Ii** |
| 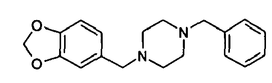 | **Ij** |
| 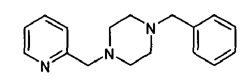 | **IIk** |
| 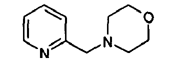 | **IIm** |
| 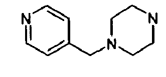 | **IIn** |
| 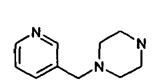 | **IIo** |
| 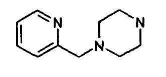 | **IIp** |
ou d'une composition comprenant au moins un composé de formules la à IIp
pour la prophylaxie et/ou le traitement de défauts de pigmentation, tels que l'hyperpigmentation, les taches de rousseur, le lentigo sénile, le lentigo solaire et le vieillissement cutané induit par l'environnement.

2. Composition comprenant au moins un dérivé de pipérazine ou de morpholine substitué de formules Ia à IIp selon la revendication 1 et au moins un ingrédient supplémentaire de soin de la peau et au moins un véhicule qui est convenable pour les applications topiques.

3. Composition selon la revendication 2, comprenant au moins un dérivé de pipérazine ou de morpholine substitué de formules Ia à IIp, **caractérisée en ce que** les compositions comprennent un ou plusieurs composés de formules I et IIp selon une quantité allant de 0,01 à 20% en poids, préférablement selon une quantité allant de 0,1 à 10% en poids, sur la base du poids total de la composition.

4. Composition selon la revendication 2 ou 3, **caractérisée en ce que** le au moins un ingrédient supplémentaire de soin de la peau est constitué par un(e) ou plusieurs antioxydants et/ou vitamines.

5. Composition selon l'une ou plusieurs parmi les revendications 2 à 4, où la composition comprend un ou plusieurs filtres UV, qui sont choisis de préférence dans le groupe constitué par le 3-(4'-méthylbenzylidène)-dlcamphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique et les sels de potassium, sodium et triéthanolamine de ceux-ci.

6. Procédé de préparation d'une composition selon la revendication 2, **caractérisé en ce qu'**un dérivé de pipérazine ou de morpholine substitué de formules Ia à IIp selon la revendication 1 est mélangé avec un véhicule convenable sur le plan cosmétique ou dermatologique.

7. Dérivé de pipérazine substitué choisi parmi les composés de formules Ia à Ie
| | |
|---|---|
| 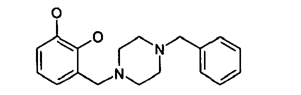 | **Ia** |
| 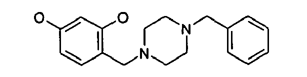 | **Ib** |
| 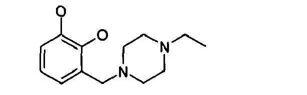 | **Ic** |
| 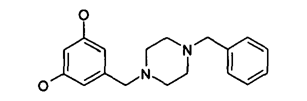 | **Id** |
| 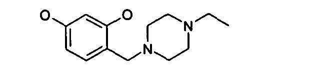 | **Ie** |
